# EUROPEAN PATENT APPLICATION

(11) **EP 2 263 624 A1**
(43) Date of publication of application: **22.12.2010**
(21) Application number: 09711801.2
(22) Date of filing: 17.02.2009
(51) Int. Cl.: A61F 13/15, A61F 5/44, A61F 13/49, A61F 13/494, A61F 13/514

(54) **ABSORPTIVE ARTICLE**

(30) Priority: 19.02.2008 JP 2008037916; 31.03.2008 JP 2008093816
(71) Applicant: Unicharm Corporation, Ehime 799-0111 (JP)
(72) Inventor: OKU, Tomomi, Kagawa 769-1602 (JP); SAKAGUCHI, Satoru, Kagawa 769-1602 (JP)
(74) Representative: Kensett, John Hinton
(86) International application number: PCT/JP2009/052645
(87) International publication number: WO 2009/104580

(57) **Abstract**

An absorptive article is provided with an absorptive body (1002) which is constructed from a liquid-permeable front surface sheet (1003), a liquid-impermeable rear surface sheet (1004), and an absorptive body (1005) mounted between the front surface sheet (1003) and the rear surface sheet (1004) and absorbing and retaining liquid and which, when worn by a user, has a rear region, a crotch region, and a belly region which extend in the longitudinal direction from the rear side to the belly side, and the absorptive article is also provided with a left and right pair of side flaps (1010) provided on opposite sides, in the lateral direction (W),of the absorptive body (1002). The lower surface of the absorptive body (1002) and those portions of the rear surface sheet (1004) which are located at the side flaps (1010) are constructed as a continuous unitized structure. That portion of each side flap (1010) which is located between a lateral edge of the side flap (1010) and that edge of the side flap (1010) which is on the absorptive body side has a high moisture-permeable region (1006) having higher moisture permeability than the lower surface of the absorptive body (1002).

## Description

### TECHNICAL FIELD

The present invention relates to an absorptive article used for sanitary napkin, pantyliner, incontinence pad, absorbent pad and so forth.

The present invention relates also to an absorptive article forming so-called pocket between an absorptive article and the skin of the user, such as diaper.

### BACKGROUND ART

There has widely been known an absorptive article having a liquid-permeable top sheet, a liquid-impermeable back sheet, and an absorber absorbing and retaining fluid. The absorber is disposed between the liquid-permeable top sheet and the liquid-impermeable back sheet. As for this sort of absorptive article, Patent Document 1 discloses a structure having the top sheet formed by a liquid-impermeable, non-woven fabric, and having a melt-blown layer provided between the absorber and the back sheet.

Patent Document 2 discloses a structure obtained by forming fold-back portions to the liquid-impermeable back sheet, and by bonding the liquid-impermeable, non-woven fabric to the fold-back portions. The fold-back portions are formed along both side edge portions in the longitudinal direction of the absorber, towards the top sheet side.

Patent Document 3 discloses a structure obtained by forming side-flap portions, extended from both side edges in the transverse direction of the absorber outwardly in the same direction, using a composite sheet provided as a separate component independent from the top sheet and the back sheet. The composite sheet is formed by making contact between an inner layer composed of a non-water-permeable sheet having a thickness of 5 µm or larger and smaller than 20 µm, and an outer layer composed of a non-woven fabric having a basis weight of 10 to 25 g/m².
Patent Document 1: Japanese Patent Application Publication No. 2001-104372
Patent Document 2: Japanese Patent Application Publication No. 2005-211131
Patent Document 3: Japanese Utility Application Publication No. H7-21020

### DISCLOSURE OF THE INVENTION

However, in the absorptive article described in Patent Publication 1, the top sheet composed of a non-woven fabric ensures only a limited range of anti-leakage function. When body fluid blocked and retained by the top sheet is applied with excessive pressure ascribable to vigorous motion of the user, the body fluid permeates through the top sheet, and leaks from the sides of top sheet. In particular, a fluid having large viscosity and small surface tension, such as loose stool, undesirably permeates and leaks through the top sheet composed of a non-woven fabric.

In the absorptive article described in Patent Document 2, the water-tightness can be improved, and leakage of excretion can successfully be prevented, by composing the back sheet by using a film having the same physical properties with the lower surface of the absorber. The film is, however, thickened at the lower surface of the absorber, because generation of pinholes, and leakage of excretion therethrough is strongly anticipated for the lower surface of the absorber, to which the body pressure is applied. For this reason, the film is consequently thickened even in other portions not so heavily applied with the body pressure. As a consequence, the lower surface of the absorber is made harder, undesirable in the texture, poor in the ventilation, and causative of stuffiness.

The absorptive article described in Patent Document 3 wastefully consumes the materials, because the absorber and side-flap portion on the sides thereof need be bonded in an overlapped manner. There is another need of arranging different materials for the absorber and for the side portions, and thereby the number of units of material increases. Equipment for manufacturing such absorptive article is therefore complicated. Bonding, by which the absorber and the side-flap portions are bound, may be broken. If broken, the excretion may undesirably leak from the absorptive article.

For these reasons, none of the conventional techniques has been successful to provide an absorptive article capable of preventing permeation and leakage, and also reducing the stuffiness.

It is therefore an object of the present invention to provide an absorptive article capable of preventing any permeation and leakage, and excellent in the ventilation.

An aspect of the present invention is summarized as an absorptive article (for example, diaper 1001A) which includes: a main absorber (main absorber 1002) composed of a liquid-permeable top sheet (top sheet 1003), liquid-impermeable back sheet (back sheet 1004), and an absorber (absorber 1005) disposed between the top sheet and the back sheet, absorbing and retaining fluid, and including a back-side region (back-side region S11), a crotch region (crotch region S12), and a belly-side region (belly-side region S13) arranged in the longitudinal direction (direction W) from the back side to the belly side in a state of wearing; and a left-and-right pair of side-flap portions (side-flap portions 1010) provided on both sides in the width direction of the main absorber. The lower surface of the main absorber and the back sheet located at the side-flap portions are joined, and each of the side-flap portions has a highly-moisture-permeable region (highly-moisture-permeable region 1006) having a larger moisture permeability as compared with the lower surface of the main absorber, between the width edge of each side-flap portion and each side edge of the absorber.

According to the present invention, the absorptive article can prevent permeation and leakage, because the lower surface of the main absorber and the back sheet on the side-flap portion side are joined. Because the side-flap portions are given as highly-moisture-permeable regions, the absorptive article can be increased in the ventilation and moisture permeability, and can avoid stuffiness. In short, the present invention can provide an absorptive article capable of preventing permeation and leakage, and excellent in the ventilation.

### BRIEF DESCRIPTION OF THE DRAWINGS

[Fig. 1] FIG. 1 is a sectional view illustrating a diaper according to embodiment 1-1 of the present invention.
[Fig. 2] FIG. 2 is an expanded view illustrating a diaper according to embodiment 1-2 of the present invention.
[Fig. 3] FIG. 3 is a sectional view taken along line A1-A1 in FIG. 2.
[Fig. 4] FIG. 4 is a sectional view taken along line B1-B1 in FIG. 2.
[Fig. 5] FIG. 5 is a sectional view illustrating a range of elevation of moisture permeability.
[Fig. 6] FIG. 6 is a sectional view illustrating a range of elevation of moisture permeability.
[Fig. 7] FIG. 7 is a sectional view illustrating a stretching machine.
[Fig. 8] FIG. 8 is a sectional view illustrating a diaper according to embodiment 1-3 of the present invention.
[Fig. 9] FIG. 9 is a sectional view illustrating a diaper according to embodiment 1-4 of the present invention.
[Fig. 10] FIG. 10 is a sectional view illustrating a diaper according to embodiment 1-5 of the present invention.
[Fig. 11] FIG. 11 is a sectional view illustrating a diaper according to embodiment 1-6 of the present invention.
[Fig. 12] FIG. 12A is a perspective view illustrating a conventional diaper, and FIG. 12B is a plan view illustrating another conventional diaper.
[Fig. 13] FIG. 13 is an expanded view illustrating a diaper according to embodiment 2-1 of the present invention.
[Fig. 14] FIG. 14 is a sectional view illustrating a diaper according to embodiment 2-1 of the present invention, taken along line A2-A2 in FIG. 13.
[Fig. 15] FIG. 15 is a sectional view illustrating a diaper according to embodiment 2-1 of the present invention, taken along line B2-B2 in FIG. 13.
[Fig. 16] FIG. 16 is a sectional view illustrating a diaper according to embodiment 2-1 of the present invention, taken along line B2-B2 in FIG. 13 in a state of wearing.
[Fig. 17] FIG. 17 is a sectional view illustrating an essential portion of a stretching machine.
[Fig. 18] FIGs. 18A and 18B illustrate a diaper according to embodiment 2-1 of the present invention, wherein FIG. 18A is a plan view illustrating a state before the center of a crotch region of the side-flap portions is stretched outward, and FIG. 18B is a plan view illustrating a state after the edge of the center portion of the crotch region of one side-flap portion is stretched outward.
[Fig. 19] FIG. 19 is a sectional view of a diaper according to embodiment 2-2 of the present invention.
[Fig. 20] FIG. 20 is a sectional view of a diaper according to embodiment 2-3 of the present invention.
[Fig. 21] FIG. 21 is an expanded view illustrating a diaper according to embodiment 2-4 of the present invention.
[Fig. 22] FIG. 22 is an expanded view of a diaper according to embodiment 2-5 of the present invention.

### BEST MODES FOR CARRYING OUT THE INVENTION

The present invention will specifically be described below, based on embodiments referring to the attached drawings.

### <First Embodiment>

### (Embodiment 1-1)

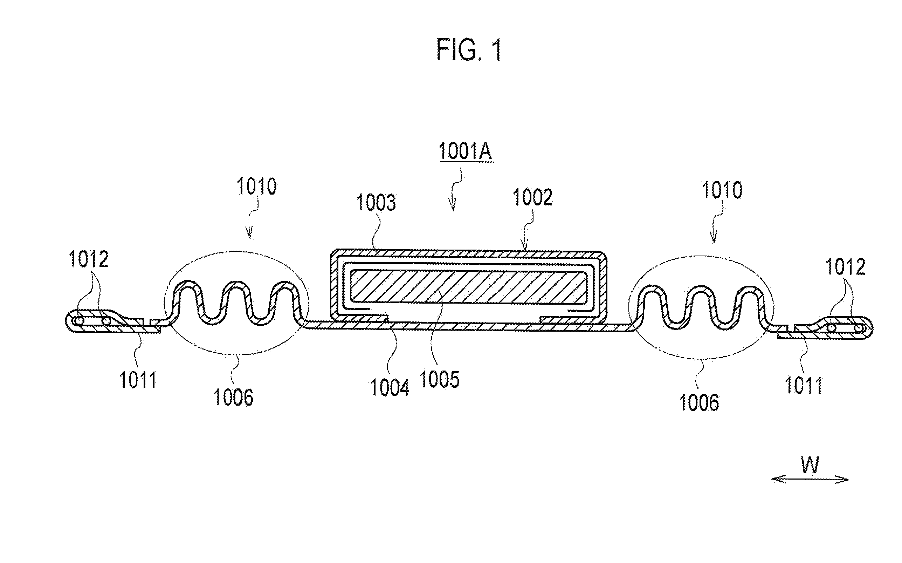

FIG. 1 is a sectional view illustrating a diaper 1001A according to embodiment 1-1 of the present invention. The diaper 1001A as an absorptive article has a main absorber 1002, and a left-and-right pair of side-flap portions 1010 provided on both sides in the width direction W of the main absorber 1002. The width direction W of the main absorber 1002 herein means the direction W normal to the longitudinal direction L ranging from the back side to the belly side.

The main absorber 1002 is composed of at least a liquid-permeable top sheet 1003, a liquid-impermeable back sheet 1004, and an absorber 1005 disposed between the top sheet 1003 and the back sheet 1004, absorbing and retaining fluid. Both edges in the width direction W of the top sheet 1003 is folded so as to wrap the absorber 1005. The fold-back portions of the top sheet 1003 and the absorber 1005 are bonded using a glue, such as hotmelt adhesive, to the back sheet 1004.

As the top sheet 1003, a hydrophilic non-woven fabric, woven fabric, perforated plastic film, perforated hydrophobic non-woven fabric and so forth may be applicable. In this embodiment, an SMS (a composite non-woven fabric of spun-bond and melt-blown fabrics, abbreviated as SMS hereinafter) having a basis weight of 10 g/m² is used as the top sheet 1003.

The absorber 1005 is a stacked material having fluid absorbing and retaining performances. The absorber 1005 is composed of a mixture containing an absorptive pulp and a super-absorbent polymer mixed therein.

The back sheet 1004 is formed by a micro-porous, liquid-impermeable film composed of a resin containing an inorganic material as a filler. More specifically, the liquid-impermeable film is formed by a polyethylene micro-porous, moisture-permeable sheet containing approximately 50 % by weight of calcium carbonate as a filler, and having a basis weight of 22.0 g/m². Besides calcium carbonate, inorganic materials such as barium sulfate may be adoptable as the filler. As the resin applicable to the moisture-permeable sheet, olefinic thermoplastic resin such as polypropylene may be applicable, besides polyethylene.

The lower surface of the main absorber 1002 and the back sheet 1004 located at the side-flap portions 1010 are joined micro-porous. In other words, the side-flap portions 1010 are formed at the portions of the back sheet 1004 stretched from the lower surface of the absorber 1005 leftward and rightward on both sides. The side-flap portions 1010 are positioned at both side edges of the absorber 1005. Each side-flap portion 1010 is provided with a highly-moisture-permeable region 1006 having a larger moisture permeability as compared with the lower surface of the main absorber 1002. In FIG. 1, the highly-moisture-permeable regions 1006 indicate regions having a large moisture permeability. The highly-moisture-permeable region 1006 are provided on both of the left and right sides of the main absorber 1002. The moisture permeability of the highly-moisture-permeable region 1006 is set to 1.3 to 2.5 times or more as large as that of the lower surface side of the main absorber 1002, but to avoid leakage of fluid. It is to be understood now that "set to avoid leakage of fluid" means that the moisture permeability of the highly-moisture-permeable region 1006 is set within a range capable of preventing the fluid from leaking.

The highly-moisture-permeable regions 1006 are formed by stretching the side-flap portions 1010 composed of a liquid-impermeable sheet in the width direction W (denoted as stretching, hereinafter). The stretching will be detailed later in embodiment 1-2.

On the outer side in the width direction of each side-flap portion 1010, an exterior sheet 1011 composed of a liquid-impermeable sheet is provided. On the side edge portion of the exterior sheet 1011, a gathered portion having elastic members 1012, in a stretched manner, is provided in the longitudinal direction. The gathered portion has a larger moisture permeability as compared with the highly-moisture-permeable regions 1006 of the side-flap portions 1010. As a consequence, a relation of the degree of moisture permeability in the diaper 1001A may be given as "lower surface of main absorber 1002<highly-moisture-permeable region 1006<gathered portion".

In thus-configured embodiment 1-1, the diaper 1001A may successfully avoid permeation and leakage of body fluid, because the back sheet 1004 composed of a liquid-impermeable sheet is provided at the lower surface of the absorber 1005.

By stretching the side-flap portions 1010 in the width direction W, the liquid-impermeable sheet (back sheet 1004) is stretched to as much as the width increased by stretching. As a consequence, micro-pores ascribable to the filler may increase, and thereby the sheet is increased in the moisture permeability. Because the improvement in moisture permeability increases the ventilation and degree of moisture permeation, the diaper 1001A may successfully avoid stuffiness in the diaper 1001A. In addition, the material is softened by the stretching, and thereby improve comfortableness felt by the user.

### (Embodiment 1-2)

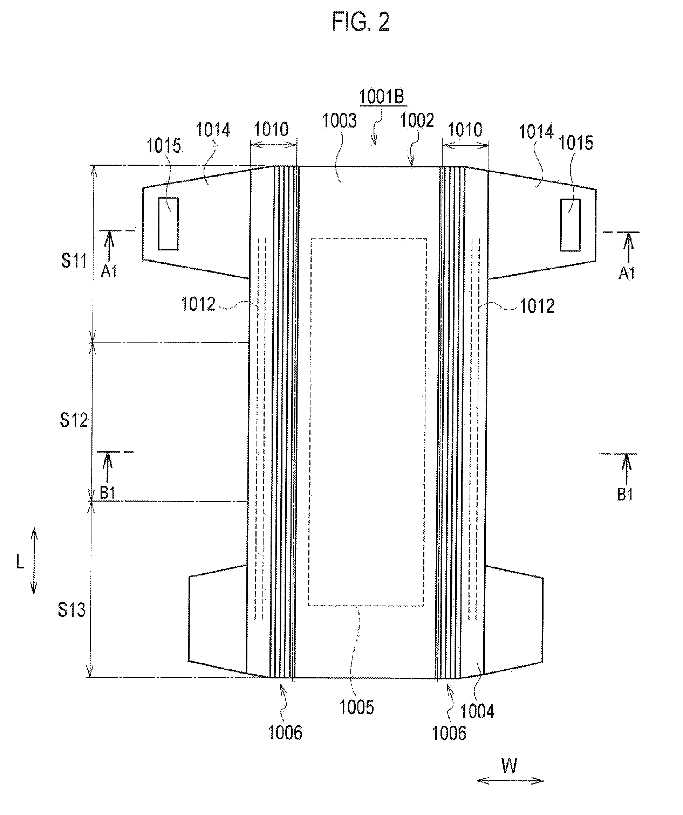

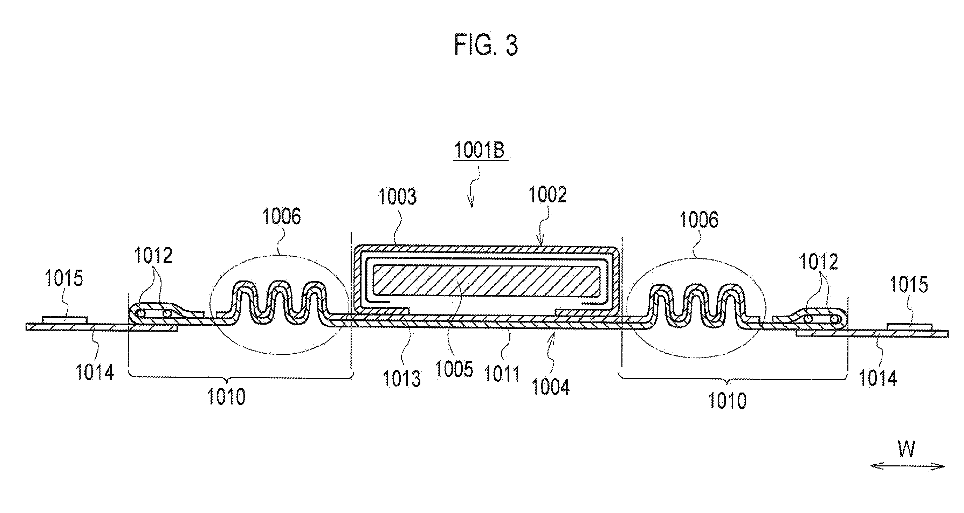

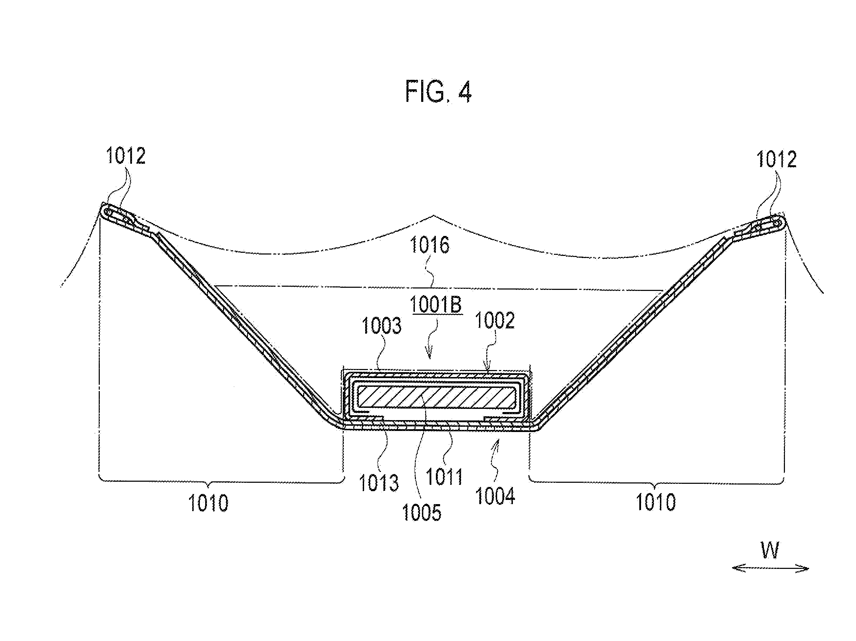

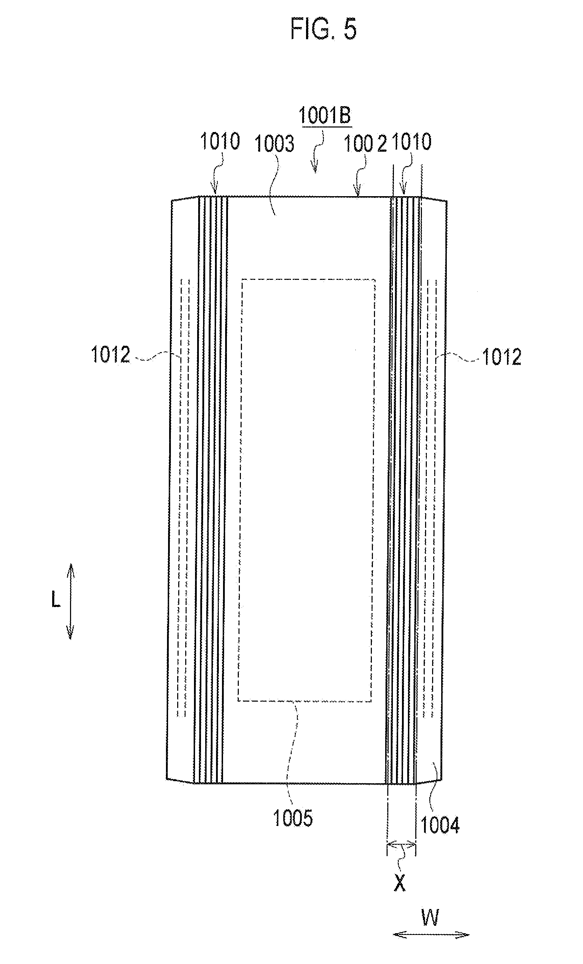

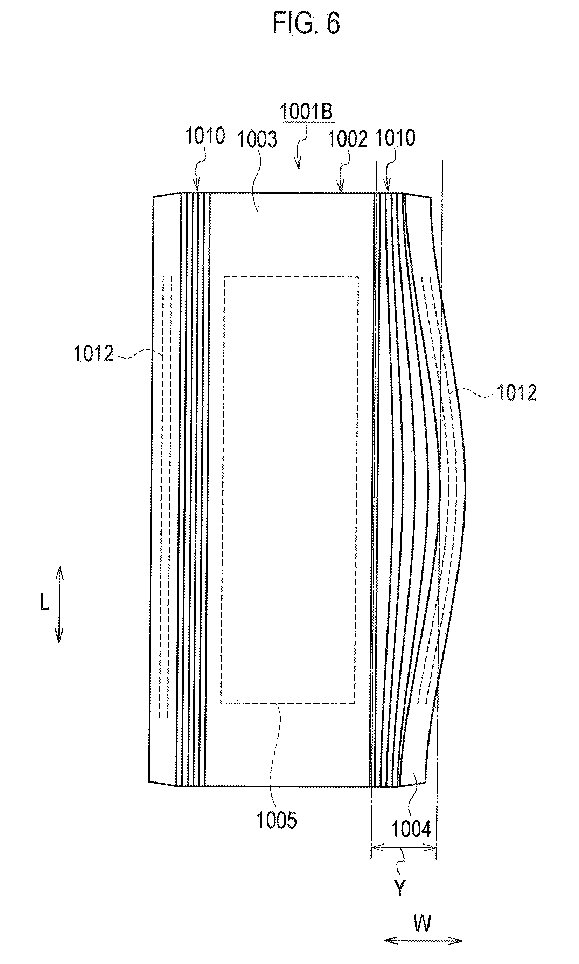

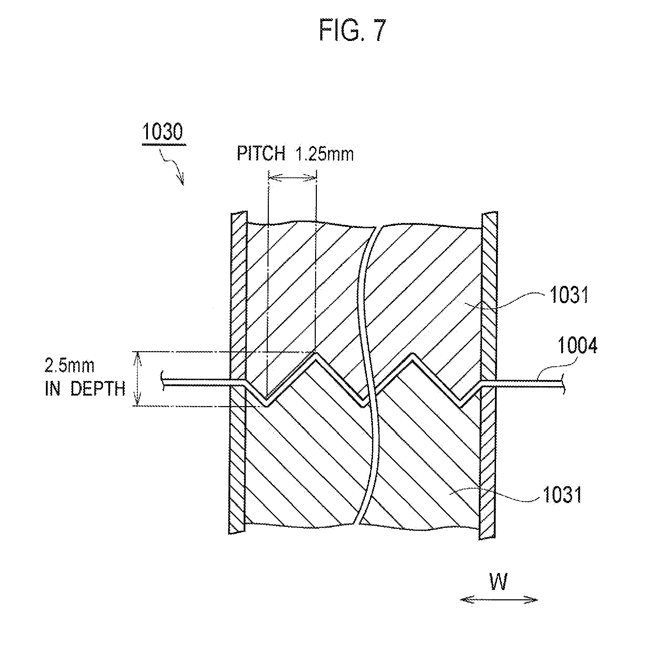

FIG. 2 to FIG. 7 illustrate an embodiment 1-2 of the present invention. FIG. 2 is an expanded view illustrating a diaper 1001B. FIG. 3 is a sectional view taken along line A1-A1 in FIG. 2. FIG. 4 is a sectional view taken along line B1-B1 in FIG. 2. FIG. 5 and FIG. 6 are plan views of the diaper 1001B illustrating ranges of elevation of moisture permeability. FIG. 7 is a sectional view of a stretching machine.

In embodiment 1-2 below, an explanation will be made mainly on aspects different from embodiment 1-1, whereas the similar explanation will not be repeated.

Similarly to as in embodiment 1-1, the diaper 1001B has the main absorber 1002, and a left-and-right pair of side-flap portions 1010 provided on both sides in the width direction W of the main absorber 1002.

The main absorber 1002 is composed of at least the liquid-permeable top sheet 1003, the liquid-impermeable back sheet 1004, and absorber 1005 absorbing and retaining fluid. The absorber 1005 is disposed between the top sheet 1003 and the back sheet 1004. The main absorber 1002 has the back-side region S11, the crotch region S12, and the belly-side region S13 in the longitudinal direction L ranging from the back side to the belly side. The main absorber 1002 covers the back side, the crotch side and the belly side of the user.

The back sheet 1004 is formed by adhering the liquid-impermeable sheet 1013 and the exterior sheet 1011. The back sheet 1004 composed of the liquid-impermeable sheet 1013 and the exterior sheet 1011 forms the side-flap portions 1010.

On the side edge portions of the side-flap portions 1010, the elastic members 1012 are disposed in a stretched manner. In the side-flap portions 1010, portions where the exterior sheet 1011 and the liquid-impermeable sheet 1013 overlap are stretched in the width direction W. The stretched portions in the side-flap portions 1010 are elevated in moisture permeability, and are given as the highly-moisture-permeable region 1006.

The top sheet 1003 is a liquid-permeable sheet. As the top sheet 1003, a hydrophilic non-woven fabric, woven fabric, perforated plastic film, and so forth, and even perforated hydrophobic non-woven fabric and so forth may be applicable. In this embodiment, an SMS having a basis weight of 10 g/m² is used as the top sheet 1003.

The liquid-impermeable sheet 1013 in the back sheet 1004 is formed by a micro-porous, liquid-impermeable film composed of a resin containing an inorganic material as a filler.

More specifically, moisture-permeable, liquid-impermeable film and a sheet obtained by bonding such liquid-impermeable film with a non-woven fabric may be adoptable as the liquid-impermeable sheet 1013. In this embodiment, a polyethylene micro-porous, moisture-permeable sheet containing approximately 50 % by weight of calcium carbonate as a filler, and having a basis weight of 22.0 g/m² is used as the liquid-impermeable sheet 1013. Inorganic materials such as calcium carbonate, barium sulfate and so forth may be adoptable as the filler. Resins adoptable to the liquid-impermeable sheet 1013 other than polyethylene include olefinic thermoplastic resin such as polypropylene.

The exterior sheets 1011 in the back sheet 1004 are liquid-impermeable sheets. In this embodiment, an SMS having a basis weight of 13 m/m² is used. The exterior sheets 1011 are disposed so as to extend from the sides of the main absorber 1002 towards the back surface. On both of the left and right side edges of the liquid-impermeable sheet 1013, the elastic members 1012 are disposed in a stretched manner. The liquid-impermeable sheet 1013 is extended out from the side portions of the absorber 1005. The liquid-impermeable sheet 1013 and the exterior sheet 1011 are bonded using a hot melt adhesive (referred to as HMA, hereinafter) or the like.

The absorber 1005 is a stack capable of absorbing and retaining fluid. The absorber 1005 is composed of a mixture having an absorbent pulp and a super-absorbent polymer mixed therein.

In the back-side region S11, waist flap portions 1014 are provided on the outer side in the width direction of the side-flap portions 1010. The waist flap portions 1014 are composed of a liquid-permeable or liquid-impermeable sheet. The waist flap portions 1014 have bindings 1015 such as mechanical binding, adhesive tape or the like, so that the absorptive article 1001B may be fixed in the belly-side portion.

In this embodiment, the exterior sheet 1011 and the liquid-impermeable sheet 1013 were adhered using an HMA or the like, to thereby from the back sheet 1004. Thereafter, two polyurethane-base elastic yarns of 470 dtex, as the elastic members 1012, were disposed on each exterior sheet 1011, stretched at a factor of stretching of 2.2, and the elastic yarns are then rolled up by each exterior sheet 1011. Then in the center region in the longitudinal direction L of each side-flap portions 1010, a portion where the exterior sheet 1011 overlaps the liquid-impermeable sheet 1013 was stretched in the width direction W. The absorber 1005 was disposed on the back sheet 1004 after the stretching. Any procedures other than those described in the above may be allowable.

Next, the highly-moisture-permeable regions 1006 provided to the side-flap portions 1010 will be explained. As illustrated in FIG. 2 and FIG. 3, the highly-moisture-permeable regions 1006 are formed to the back sheet 1004 composed of the liquid-impermeable sheet 1013 and the exterior sheet 1011. Each highly-moisture-permeable region 1006 is provided between one edge in the width direction W of each side-flap portion 1010 and each side edge of the absorber 1005. The highly-moisture-permeable regions 1006 are formed by stretching the back sheet 1004.

FIG. 5 illustrates portions to be stretched. For the diaper 1001B typically having an overall length of 470 mm in the longitudinal direction L, the back sheet 1004 is stretched outwardly in the width direction over a range of 20 mm, while assuming a position 5 mm outwardly away from each side portion of the absorber 1005 in the width direction W as a reference position. For the longitudinal direction L, the absorber 1005 is stretched over the overall length thereof. The range of the portion to be stretched is not a limitative factor, because the design may be subject to change depending on the length of diaper, the width of the absorber 1005 and so forth. For open-type diapers or pants-type diapers, the highly-moisture-permeable regions 1006 are preferably provided over the entire length of the portion between the side-flap portions disposed at the front and rear in the longitudinal direction L (between the front and rear panels for the pants-type diapers).

The exterior sheet 1011 and the liquid-impermeable sheet 1013 are adhered using an HMA or the like, to thereby produce the back sheet 1004, and the back sheet 1004 is then stretched using a stretching machine 1030 illustrated in FIG. 7.

As illustrated in FIG. 7, the stretching machine 1030 has a pair of stretching blade array portions 1031 opposed and engageable with each other. Each of the pair of stretching blade array portions 1031 has a depth of blade of 2.5 mm, a pitch of upper and lower blades of 1.25 mm, and the number of upper and lower blades of 17. A portion of the side-flap portions 1010 is stretched, while setting the temperature of the stretching blade array portion 1031 to 90°C. By the stretching, the highly-moisture-permeable regions 1006 are stretched by a factor of 1.8 as compared with the width before the stretching. More specifically, the highly-moisture-permeable regions 1006 are elevated in the moisture permeability by a factor of approximately 1.65, from the average moisture permeability of 1935 (Pa·24hrs) before stretching up to 3191 (Pa·24hrs) after stretching (see Table 1).

In the process of stretching, heating of the blades are soften the back sheet 1004, more readily reduce the basis weight, and make pinholes less likely to produce. An appropriate temperature of heating may preferably set 20 to 30°C lower than the melting points of the materials. Heating at above the melting points of the materials of the liquid-impermeable sheet 1013 may undesirably melt and break the liquid-impermeable sheet 1013.

The back sheet 1004 is obtained by coating an HMA over a 15-mm-diameter area at a basis weight of 5 g/m² using a spiral spray, and bonding the moisture-permeable film (liquid-impermeable sheet 1013) having a basis weight of 22.0 g/m², with the liquid-permeable SMS (exterior sheet 1011) having a basis weight of 13 g/m². Table 1 shows results of the stretching.

**Table 1**

| | | n=1 | n=2 | n=3 | Average |
|---|---|---|---|---|---|
| Before elevated in moisture permeability (Before stretching) | Initial weight (g) | 71.39 | 64.12 | 66.15 | |
| | Weight after allowed to stand for 24 hrs (g) | 65.53 | 59.06 | 60.67 | |
| | Difference in weight (g) | 5.85 | 5.06 | 5.49 | |
| | Moisture permeability (Pa·24 hrs) | 2071.4 | 1791.6 | 1941.8 | 1934.9 |
| After elevated in moisture permeability (After stretched) | Initial weight (g) | 66.65 | 67.96 | 67.41 | |
| | Weight after allowed to stand for 24 hrs (g) | 57.58 | 59.10 | 58.29 | |
| | Difference in weight (g) | 9.07 | 8.86 | 9.11 | |
| | Moisture permeability (Pa·24 hrs) | 3210.8 | 3136.6 | 3224.8 | 3190.8 |

FIG. 5 and FIG. 6 illustrate ranges of stretching (elevating the moisture permeability). The width of the side-flap portions 1010 to be stretched is defined as X, ranging from each side edge of the absorber 1005 up to a portion close to the inner side of the elastic member 1012 disposed closest to the absorber 1005 in each side-flap portion 1010, in an extended view of the diaper 1001B (FIG. 5). In a state of stretching the crotch region of each side-flap portion 1010, the width ranging from the side edge of the absorber 1005 up to a portion close to the inner side of the elastic member 1012 disposed closest to the absorber 1005 in each side-flap portion 1010, is defined as Y (FIG. 6). Width Y preferably falls in the range of 1.5 to 3.0 times as large as width X.

If width Y falls short of 1.5 times as large as width X, the width of stretching of the highly-moisture-permeable regions 1006 is too small to ensure sufficient height of the side-flap portions 1010 arisen when the diaper is worn by the user. If so, there may be a risk of producing a gap between the skin and the portions having the elastic members 1012 disposed therein, and causing leakage of excretion through the gap.

If width exceeds 3.0 times as large as width X, the width of stretching of the highly-moisture-permeable regions 1006 is large enough to excessively elevate the moisture permeability due to stretching. As a consequence, the materials may become more likely to be damaged, and raises a higher risk of fractures of the materials, such as breakage and piercing. If the materials got fractured, there may be a higher risk of leakage of excretion through the fractured portion.

The moisture permeability of the stretched regions (highly-moisture-permeable regions 1006) after stretching is preferably 1.3 to 2.5 times or more as large as the moisture permeability before stretching. In this context, the moisture permeability before stretching more specifically means the moisture permeability of the lower surface side of the absorber. If difference between the moisture permeabilities obtained before and after stretching may fall short of 1.3 times, no substantial change is made in the moisture permeability as compared with the status before stretching, only to raise an insufficient effect of reducing stuffiness in the diaper.

The liquid-impermeable sheet 1013 suitable for stretching may be any of those having a tensile rupture strength of 750% or larger. Films having a tensile rupture strength of smaller than 750% may be likely to produce pinholes during stretching. Production of pinholes may be causative of leakage.

In the above-described embodiment, by stretching of the side-flap portions 1010, the back sheet 1004 is stretched to a degree corresponding to the width of stretching. As a consequence, the micro-pores ascribable to the filler may be enlarged, and thereby the stretched highly-moisture-permeable regions 1006 are elevated in the moisture permeability. The highly-moisture-permeable regions 1006 are, therefore, reduced in the basis weight of material as compared with the un-stretched regions, and elevated in the moisture permeability (so-called enhancement of moisture permeation). The materials composing the highly-moisture-permeable regions 1006 may be softened by the enhancement of moisture permeation. Moreover, the diaper 1001b may keep the level of height of disposition of the elastic members 1012 higher, to a degree corresponding to the width of stretching, than the surface of the fluid-permeable sheet. As a consequence, as illustrated in FIG. 4, the diaper 1001b may ensure a large pocket 1016 catching body fluid, as compared with any unstretched diapers.

As has been described in the above, according to embodiment 1-2, the diaper 1001b is configured by bonding the liquid-impermeable sheet 1013 and the exterior sheet 1011 to form the back sheet 1004, and by disposing the highly-moisture-permeable regions 1006 with enhanced moisture permeation, in the side-flap portions 1010 of the back sheet 1004, along the side edges of the absorber 1005. Accordingly, the diaper 1001b is given with a structure having a film material disposed in the side-flap portions 1010, improved in the water-proofness, and can prevent leakage of body fluid. Moreover, the diaper 1001b may successfully avoid the internal stuffiness causative of skin troubles, by virtue of improvement in the ventilation and moisture permeability as an effect of enhanced moisture permeation.

Still additionally, according to embodiment 1-2, softened regions are formed by stretching, in the side-flap portions 1010 located on the inner surface of the diaper 1001b. By virtue of softness of the inner surface of the diaper 1001b, the diaper 1001b may reduce uncomfortable feeling of wearing as compared with any unstretched diapers.

The softness of the inner surface of the diaper 1001b also enhances stretchability of the elastic members 1012, and improves rise-up of the side-flap portions 1010 up above the diaper. The diaper 1001b may consequently be improved in the fitness to the body, and may be reduced in the risk of leakage of excretion.

The diaper 1001b has no highly-moisture-permeable region disposed on the lower surface of the absorber 1005, where the body pressure is most heavily applied, and instead the lower surface of the absorber 1005 is elevated in the water-proofness as compared with the highly-moisture-permeable regions 1006. Accordingly, even after a long duration of use, the diaper 1001b may prevent excretion from permeating through the lower surface of the absorber 1005, or moisture from leaking therethrough, and successfully prevent underwear and bedding from getting wet.

According to embodiment 1-2, the diaper 1001b may be given with different effects in the side-flap portions 1010 and in the lower surface of the absorber 1005, only by disposing a single material continuous in the width direction W. More specifically, the side-flap portions 1010 are excellent in the ventilation and moisture-permeability, and the lower surface of the absorber 1005 is excellent in the water-proofness.

In this embodiment, the liquid-impermeable sheet 1013 and the exterior sheet 1011 composing the back sheet 1004 is preferably adhered with the aid of an HMA using a spiral spray. Adhesion with the aid of an HMA using a spiral spray enables local adhesion between the materials, and may keep a small density of area of adhesion. Low density of area of adhesion is advantageous in that elongation of the liquid-impermeable sheet 1013 in the process of stretching is less likely to be inhibited by the exterior sheet 1011, so that fractures of the materials during stretching may be less likely to occur. In contrast, when the density of area of adhesion between the materials to be bonded is high, such as obtained by curtain coating of HMA, elongation of the liquid-impermeable sheet 1013 in the process of stretching may be inhibited by the exterior sheet 1011. In other words, stretching under a high density of area of adhesion may raise a higher risk of fracture of the materials. In the process of adhesion using an HMA, the HMA may be used in a state softened by preheating.

The elastic members 1012 may be disposed to anywhere in the unstretched regions in the side-flap portions 1010 of the back sheet 1004. The number of provision of the elastic members 1012 is not specifically limited, and may be variable depending on functions and design of the diaper. For the case where a plurality of elastic members 1012 are disposed in each side-flap portion 1010 to thereby form a plane, and such plane is brought into contact with the user, the elastic member 1012 disposed closest to the absorber 1005 may preferably be disposed in the non-highly-moisture-permeable region, at around the boundary between each highly-moisture-permeable region 1006 and non-highly-moisture-permeable region.

According to this configuration, boundaries between the highly-moisture-permeable regions 1006 and the non-highly-moisture-permeable regions may more readily be given as creases, by virtue of difference in rigidity between the highly-moisture-permeable regions 1006 and the non-highly-moisture-permeable regions. Because the non-highly-moisture-permeable regions are lifted up along the creases, the regions of the side-flap portions 1010 having the elastic members 1012 disposed therein are brought into fitting with the skin without producing wrinkles. Accordingly, the diaper 1001b may be improved in the sealing performance.

In embodiments 1-3 to 1-6 below, explanations will be made mainly on aspects different from those in embodiments 1-1 and 1-2, whereas similar explanations will not be repeated.

### (Embodiment 1-3)

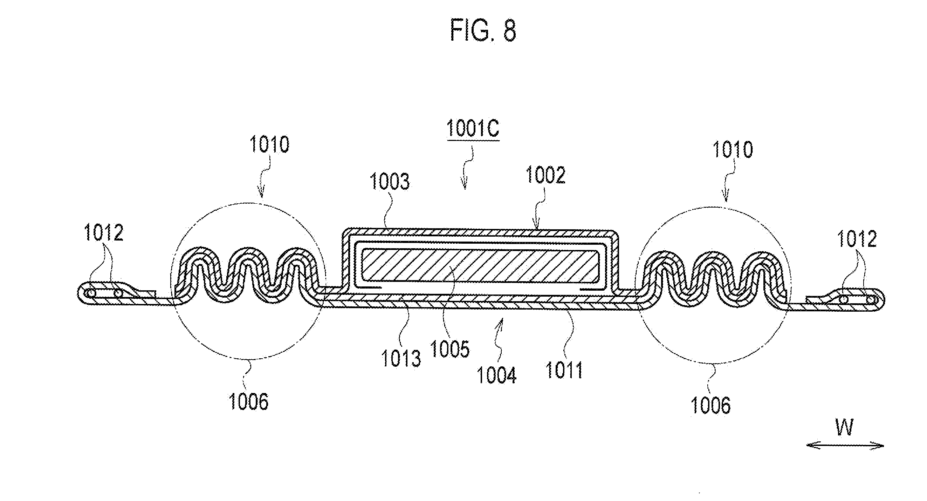

FIG. 8 illustrates embodiment 1-3 of the present invention. In a diaper 1001C of this embodiment, the back sheet 1004 is formed by adhering the liquid-impermeable sheet 1013 and the exterior sheet 1011. The top sheet 1003 covers the absorber 1005, and extends sidewards. The top sheet 1003 uses a hydrophilic fluid-permeable sheet. The top sheet 1003, the liquid-impermeable sheet 1013 and the exterior sheet 1011 are adhered. The adhered portions are stretched (elevated in the moisture permeability). In other words, the highly-moisture-permeable regions 1006 are formed in the liquid-impermeable sheet 1013, the exterior sheet 1011, and the top sheet 1003.

Thus-configured diaper 1001C may guide body fluid once flown from the side portions of the absorber 1005 towards the highly-moisture-permeable regions 1006 of the side-flap portions 1010, back into the absorber 1005, with the aid of the hydrophilic fluid-permeable sheet (top sheet 1003).

### (Embodiment 1-4)

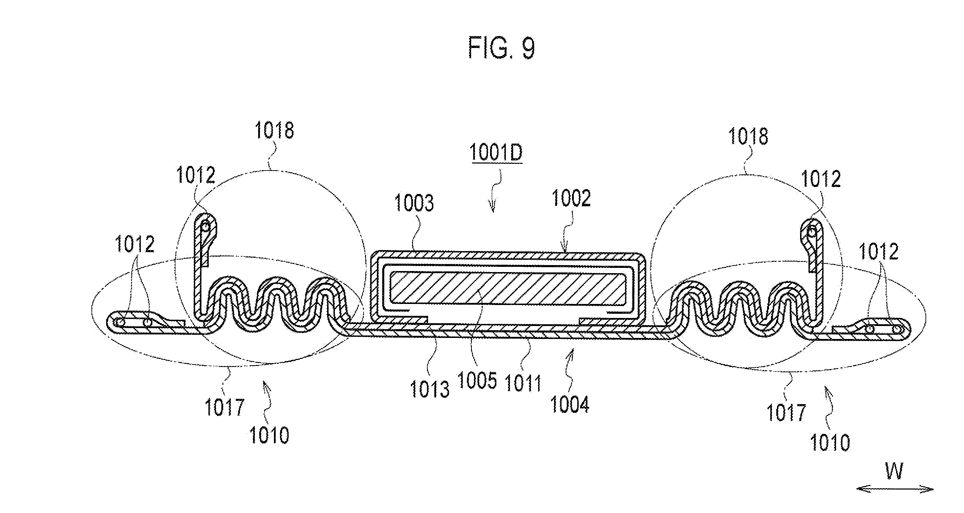

FIG. 9 illustrates embodiment 1-4 of the present invention. In a diaper 1001D of this embodiment, the side-flap portions 1017, 1018 are disposed in two rows on each side, and each of two rows of side-flap portions 1017, 1018 are stretched (elevated in the moisture permeability). Because two rows of side-flap portions 1017, 1018 are disposed on the side portions of the absorber 1005, the diaper 1001D may doubly express its anti-leakage function. Because the liquid-impermeable sheet 1013 composing the back sheet 1004 is covered with the side-flaps 1018, the diaper 1001D may be improved also in the touch.

### (Embodiment 1-5)

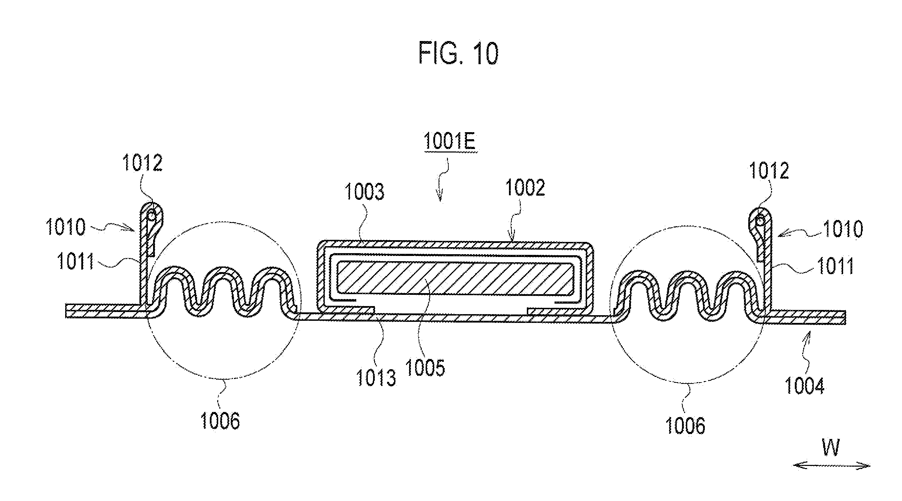

FIG. 10 illustrates embodiment 1-5 of the present invention. In the diaper 1001E of this embodiment, the side-flap portions 1010 are formed so as to incline inwardly, and similarly stretched (elevated in the moisture permeability). In thus-configured diaper 1001E, the surface of the absorber 1005 is covered with the side-flap portions 1010, so that the area of the absorber 1005 brought into contact with the skin may be reduced, and thereby excretion is less likely to return back to the skin.

### (Embodiment 1-6)

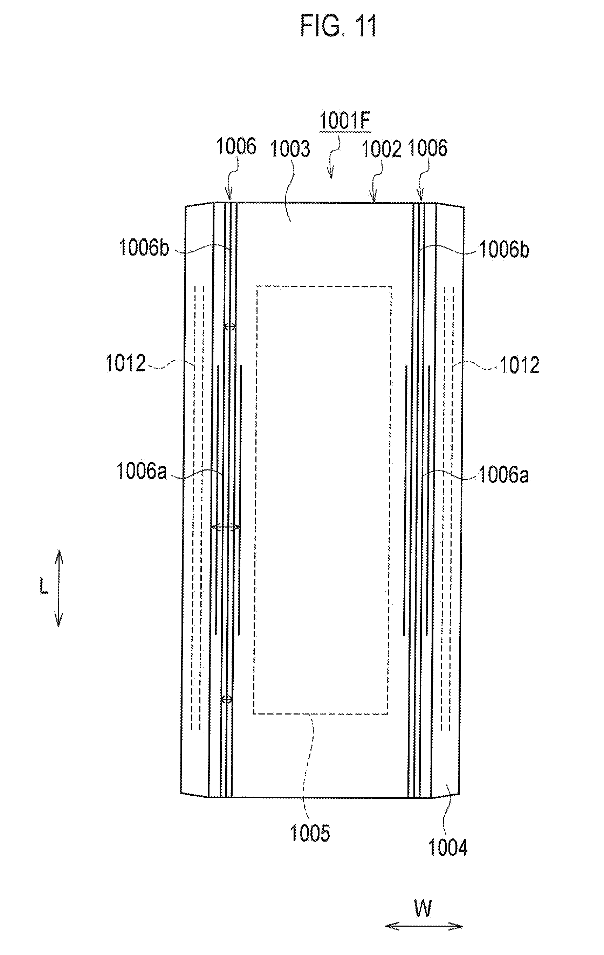

FIG. 11 illustrates embodiment 1-6 of the present invention. In a diaper 1001F of this embodiment, the moisture permeability is elevated over a region more widely in the center portion 1006a, in the longitudinal direction L, of each highly-moisture-permeable region 1006, than in the end portions 1006b thereof. Because the moisture permeability is elevated over a region more widely in the center portion 1006a than in the end portions 1006b, the width of the pocket becomes wider in the center portion 1006a than in the end portions 1006b. In other words, the diaper 1001F may ensure a large capacity in the crotch region where excretion is most likely to stay. The diaper 1001F may, therefore, suppress the leakage therefrom.

In the process of accomplishing this embodiment, the height of indentation blades is altered between the end portions 1006b and the center portion 1006a. More specifically, by setting the height of the indentation blades higher in the center portion 1006a than in the end portions 1006b, the center portion 1006a may more strongly be elevated in the moisture permeability than the end portions 1006b. In addition, by elevating the moisture permeability in the center portion 1006a over a larger area in the width direction W than in the end portions 1006b, that is, by altering the area, in which the moisture permeability is enhanced, between the center portion 1006a and the end portions 1006b, the center potion 1006a may be elevated in the moisture permeability to a larger degree than in the end portions 1006b.

### (Other First Embodiment)

In this embodiment, the diaper has the portions elevated in the moisture permeation (stretched portions) on the sides of the absorber 1005, but the diaper is not limited to those of open-type. The diaper may be a pants-type, having the body thereof folded double at the center in the longitudinal direction L, and both portions on the back side and the belly side are bound and fixed. This type of diaper may ensure a large capacity in the crotch portion where excretion is most likely to stay, so that leakage from the diaper may be suppressed.

The present invention is not limited to the above-described diaper, but may be adoptable to other absorptive articles such as sanitary napkin and absorbent pad.

In this embodiment, a polyethylene film containing approximately 50 % by weight of calcium carbonate as a filler is adopted to the liquid-impermeable sheet 1013 used for the back sheet 1004. This sort of film adoptable herein may also be a film having micro-pores formed by stretching in the process of manufacturing, and thereby expressing moisture permeability before being elevated in the moisture permeability in the processing of diaper.

Similarly, this sort of film adoptable herein may also be a non-moisture-permeable polyethylene film containing 20 to 70 % by weight of calcium carbonate as a filler, and having a basis weight of 15 to 40 g/m², which is substantially not stretched in the process of manufacturing. In this case, the back sheet 1004 is elongated only to as much as the width of stretching in the processing of diaper, wherein the film will have micro-pores ascribable to the filler formed therein, and will have moisture permeability.

Because the film has no micro-pores at the lower surface of the absorber 1005, where the body pressure is most heavily applied, the diaper may keep the water-proofness to a higher level, and may effectively prevent fluid from permeating through the liquid-impermeable sheet 1013.

In this embodiment, a moisture-permeable film is used as the film. Resins adoptable to the moisture-permeable film may be exemplified by polyethylene resin, polypropylene resin, fluorine-containing resin, which may be formed into a sheet of as thick as 20 to 40 µm by film-making and stretching under fusion.

This application is based upon and claims the benefit of priority from the prior Japanese Patent Application No. JP 2008-093816, filed on March 31, 2008; the entire contents of which are incorporated herein by reference.

### <Second Embodiment>

### [BACKGROUND ART]

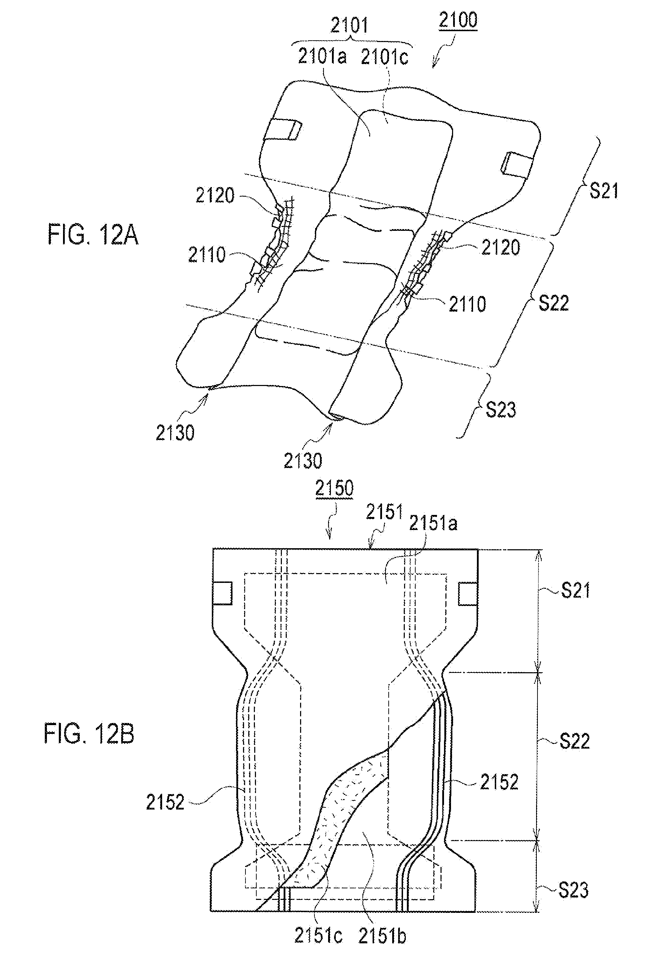

There has been known still another diaper in which a pocket is formed between the user's skin and the crotch region of the diaper (for example, Patent Document 4. As illustrated in FIG. 12A, a diaper 2100 has a main absorber 2101 composed of a liquid-permeable top sheet 2101a, a liquid-impermeable back sheet (not illustrated), and an absorber 2101c absorbing and retaining fluid. The absorber 2101c is disposed between the top sheet 2101a and the back sheet (not illustrated). The main absorber 2101 includes a back-side region S21, a crotch region S22, and a belly-side region S23 arranged in the longitudinal direction from the back side to the belly side in a state of wearing, and covers the back side and the belly side of the user, centered round the crotch. On both sides in the width direction of the main absorber 2101, side-flap portions 2110 are provided by extending the top sheet 2101a and the back sheet (not illustrated). On the outer edge of the crotch region S22 of each side-flap portion 2110, an elastic member 2120 is provided in the longitudinal direction, under a stretched state. In the belly-side region S23 of each side-flap portion 2110, a fold-back portion 2130 is provided.

When the user wears the conventional diaper 2100, the outer periphery of each side-flap portion 2110 comes into close contact with the leg-opening portion of the user as a result of shrinkage of each elastic member 2120, and a space, or so-called pocket, is formed on the crotch side of the user, because the width dimension of each side-flap portion 2110 in the crotch region S22 is larger than the width dimension in the belly-side region S23. By virtue of the pocket, urine, feces and so forth excreted from the user can surely be caught, without causing side leakage.

Still another known conventional diaper 2150 is disclosed in Patent Document 5. The diaper 2150 has, as illustrated in FIG. 12B, a main absorber 2151 composed of a liquid-permeable top sheet 2151a, a liquid-impermeable back sheet 2151b, and an absorber 2151c absorbing and retaining fluid. The absorber 2151c is disposed between the top sheet 2151a and the back sheet 2151b. The main absorber 2151 has the back-side region S21, the crotch region S22 and the belly-side region S23 arranged in the longitudinal direction from the back side to the belly side of the user, and covers the back side and the belly side of the user, centered round the crotch. On both sides in the width direction of the main absorber 2151, a pair of elastic members 2152 are provided, under a stretched state. Each elastic member 2152 is provided so as to swell outward at the crotch region S22. Accordingly, the distance between the pair of elastic members 2152 is set smaller in the back-side region S21 and the belly-side region S23, and larger in the crotch region S22.

When the user wears the conventional diaper 2150, the outer peripheries of the crotch region S22 come into close contact with the leg-opening portion of the user, as a result of shrinkage of the elastic members 2152, and a space, or so-called pocket, is formed in the crotch side of the user, because the distance between the pair of elastic members 2152 is wider in the crotch region S22 of the main absorber 2151. By virtue of the pocket, similarly to as in the above-described conventional example, urine, feces and so forth excreted from the user can surely be caught, without causing side leakage.

Patent Document 4: Japanese Patent Application Publication No. S62-199802
Patent Document 5: Japanese Patent Application Publication No. H4-242645

### [DISCLOSURE OF THE INVENTION]

However, these two last above-described conventional examples, characteristically forming the pocket to the crotch region S22 in a state of wearing, need additional materials corresponding to the portions widened in the width direction of the diapers 2100, 2150, as compared with those having no pocket formed therein.

The former of these two last above-described conventional examples is also disadvantageous in that processes in the manufacturing is labor-consuming and consequently raises the cost, because of need of providing the fold-back portions 2130 to the individual side-flap portions 2110.

The latter of these two last above-described conventional examples is also disadvantageous again in that processes in the manufacturing is labor-consuming and consequently raises the cost, because of need of providing the pair of elastic members 2152 in a curved pattern.

It is therefore an object of the present invention to provide an absorptive article characteristically forming so-called pocket on the crotch side of the user, capable of reducing consumption of the materials, simplifying processes in the manufacturing, and reducing the cost.

An aspect of the present invention, aimed at accomplishing the above-described object, is summarized as an absorptive article (for example, diaper 2001A), which include: a main absorber (main absorber 2002) composed of at least a liquid-permeable top sheet (top sheet 2003), a liquid-impermeable back sheet (back sheet 2004), and an absorber (absorber 2005) disposed between the top sheet and the back sheet, absorbing and retaining fluid, and including a back-side region (back-side region S21), a crotch region (crotch region S22), and a belly-side region (belly-side region S23) arranged in the longitudinal direction from the back side to the belly side in a state of wearing; a left-and-right pair of side-flap portions (side-flap portions 2010) provided on both sides in the width direction of the main absorber; and a pair of elastic members (for example, elastic member "a") provided to at least the crotch region of the individual side-flap portions, stretched in the longitudinal direction. The absorptive article further include a crotch-side stretched region (for example, crotch-side stretched region 2013) stretched in the width direction, provided to the side-flap portion in the crotch region, between the main absorber and the outermost elastic member.

According to the present invention, in the absorptive article in a state of wearing, the outer periphery of each side-flap portion comes into contact with the leg-opening portion of the user as a result of shrinkage of each elastic member, and so-called pocket is formed at the crotch region of the user, because the expandable width dimension of the crotch region of each side-flap portion is set larger than the width dimensions of the side-flap portions in the back-side region and the belly-side region, by a contribution of the pair of crotch-side stretched regions. Since the individual crotch-side stretched regions are provided by stretching the crotch region of the individual side-flap portions in the width direction, the crotch-side stretched regions may be formed using a material having only the same width with those in a diaper used without forming so-called pocket in the crotch region. Additionally, the pocket may be formed on the crotch side of the user, without providing the fold-back portions to the individual side-flap portions, and without providing the elastic members in a curved pattern to the individual side-flap portions. As a consequence, consumption of the materials, simplification of processes in the manufacturing, and reduction in the cost of manufacturing may be accomplished.

Because the individual side-flap portions, covering the leg-opening portion close to the crotch of the user, are given as thin and soft crotch-side stretched regions, the side-flap portions may raise still other advantages in that they may be less uncomfortable in wearing, they may ensure high compliance with motion of the legs of the user, they may easily be risen-up from the main absorber, and they may be brought into close contact with the leg-opening portion without too much tightening the outer peripheries thereof.

### [BEST MODES FOR CARRYING OUT THE INVENTION]

The present invention will specifically be described below, based on embodiments referring to the attached drawings.

### (Embodiment 2-1)

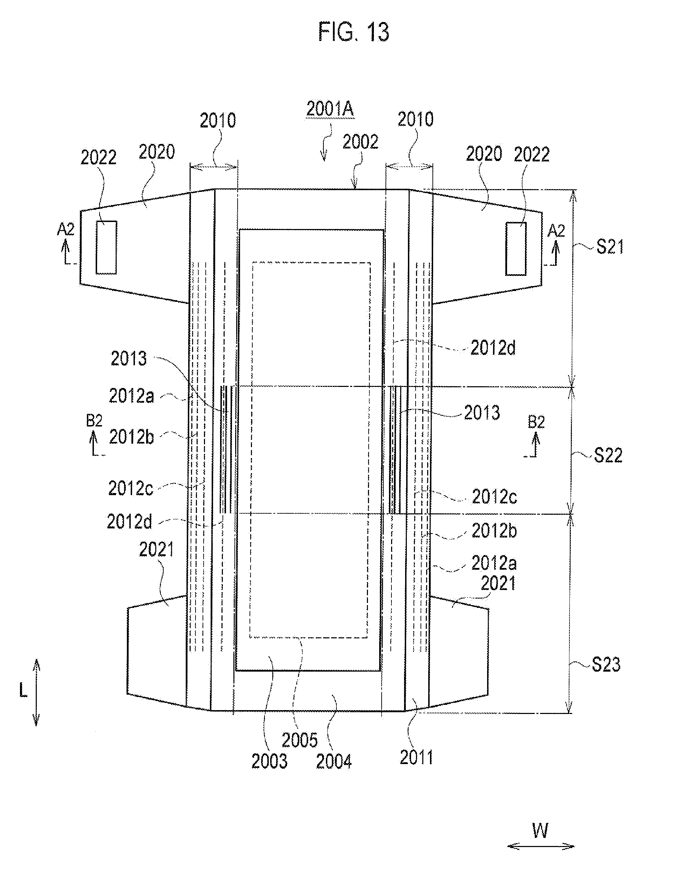

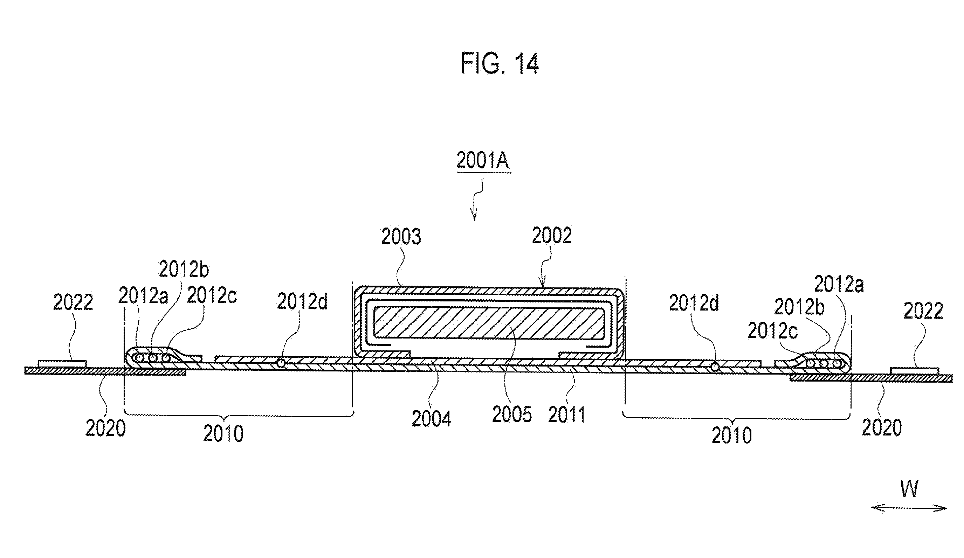

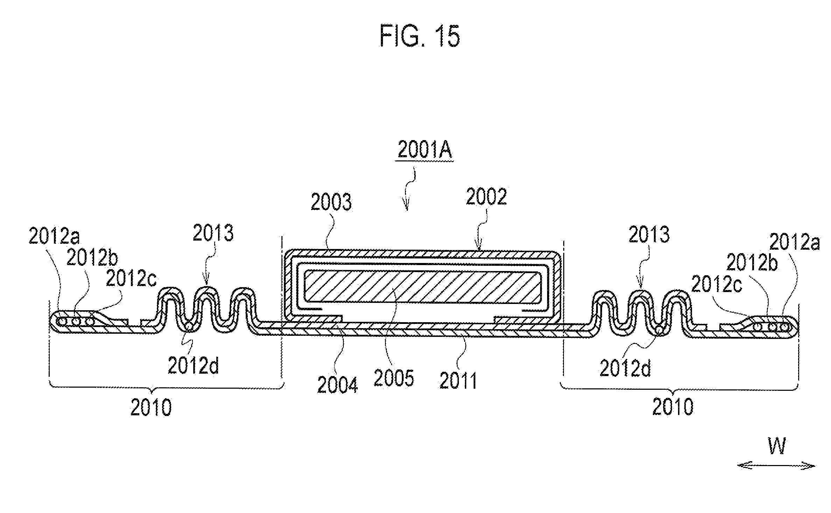

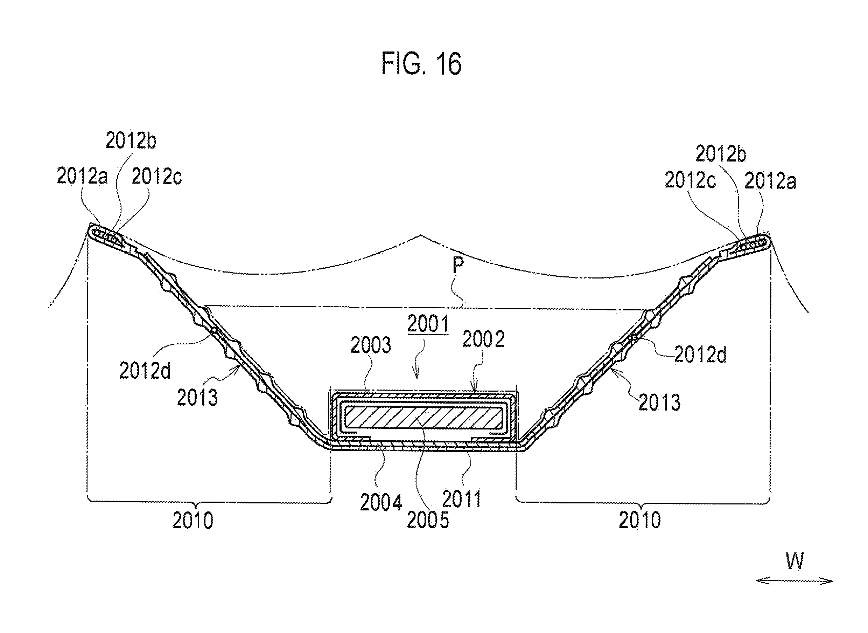

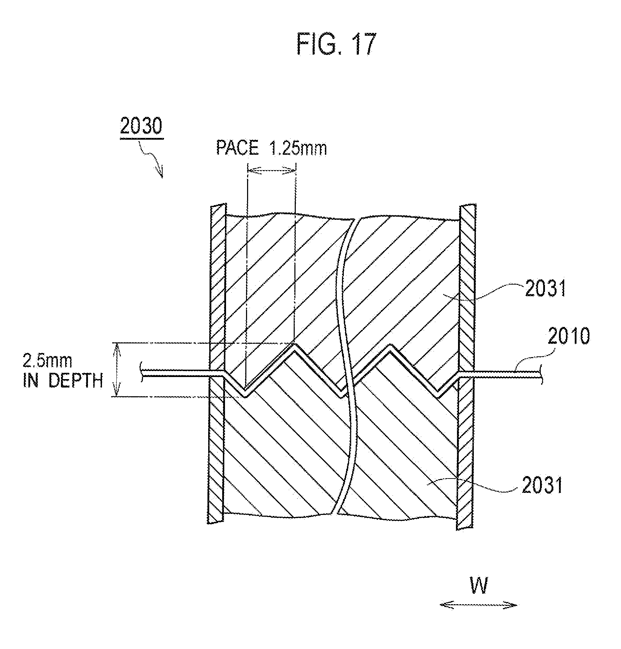

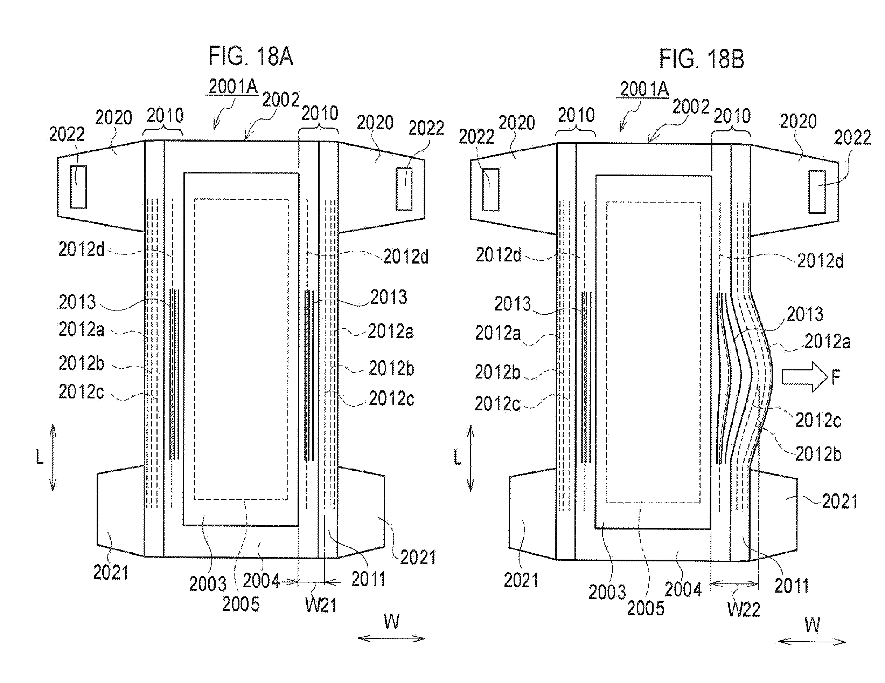

FIG. 13 to FIG. 18B illustrate embodiment 2-1 of the present invention. FIG. 13 is an expanded view illustrating a diaper 2001A. FIG. 14 is a sectional view taken along line A2-A2 in FIG. 13. FIG. 15 is a sectional view taken along line B2-B2 in FIG. 13. FIG. 16 is a sectional view of the diaper 2001A in a state of wearing. FIG. 17 is a sectional view illustrating an essential portion of a stretching machine. FIG. 18A is a plan view illustrating a state before the center of the crotch region of the side-flap portions is stretched outward. FIG. 18B is a plan view illustrating a state after the edge of the center portion of the crotch region of one side-flap portion is stretched outward.

As illustrated in FIG. 13 to FIG. 15, the diaper 2001A as the absorptive article has the main absorber 2002, a left-and-right pair of side-flap portions 2010 provided on both sides of the main absorber 2002, a pair of back-side waist flap portions 2020 provided on both ends in the width direction W on the back side of the pair of side-flap portions 2010, and a pair of belly-side waist flap portion 2021 provided on both ends in the width direction W on the belly side of the pair of side-flap portions 2010.

The main absorber 2002 is composed of at least a liquid-permeable top sheet 2003, a liquid-impermeable back sheet 2004, and an absorber 2005 disposed between the top sheet 2003 and the back sheet 2004, and absorbing and retaining fluid. The diaper 2001A in which a back-side and a belly-side are defined and a back-side region region S21, a crotch region S22, and a belly-side region S23 are sequentially provided along with a longitudinal direction from the back-side toward the belly-side. The main absorber 2002 has the back-side region S21, the crotch region S22 and the belly-side region S23 arranged in the longitudinal direction L from the back side to the belly side of the user, and covers the back side, the crotch side and the belly side of the user.

The top sheet 2003 is folded at both end portions thereof in the width direction W, so as to wrap the absorber 2005. Portions of the top sheet 2003 thus folded and the absorber 2005 are bonded to the back sheet 2004 using a glue such as hotmelt adhesive. The top sheet 2003 may be formed using hydrophilic non-woven fabric, woven fabric, perforated plastic film, perforated hydrophobic non-woven fabric, and spun-bond/melt-blown/spun-bond, non-woven fabric (abbreviated as SMS, hereinafter), having a basis weight of 10 g/m².

The back sheet 2004 is formed using a moisture-permeable or non-moisture-permeable film, a sheet obtained by bonding such film with a non-woven fabric, and so forth. The absorber 2005 is a stack capable of absorbing and retaining fluid. The absorber 2005 is formed by mixing an absorbent pulp and a super-absorbent polymer.

The pair of side-flap portions 2010 are composed of the back sheet 2004 extended from both sides in the width direction W of the main absorber 2002, and a liquid-impermeable exterior sheet 2011. The exterior sheet 2011 is disposed stacked on the back surface of the main absorber 2002 and the back sheet 2004.

The back sheet 2004 and the exterior sheet 2011 are bonded using a glue such as hotmelt adhesive. The exterior sheet 2011 is formed to have a width dimension larger than that of the back sheet 2004.

Over the entire range of the crotch region S22 of the individual side-flap portions 2010, and parts of the back-side region S21 and the belly-side region S23 continued from the crotch region S22, four rows of elastic members 2012a to 2012d are provided in the longitudinal direction L, under a stretched state.

The outer three rows of elastic members 2012a to 2012c are provided to both side portions of the exterior sheet 2011 which is formed longer in the width direction W than the back sheet 2004. The innermost elastic member 2012d is arranged nearly at the center position between each side edge of the main absorber 2002 and the outermost elastic member 2012a y. The elastic member 2012d is provided between the exterior sheet 2011 and the back sheet 2004. The individual elastic members 2012a to 2012d are typically composed of polyurethane-base elastic yarn.

In the crotch region S22 of each side-flap portion 2010, and between the main absorber 2002 and the outermost elastic member 2012a, there are provided the crotch-side stretched region 2013 stretched in the width direction W. More specifically, each crotch-side stretched region 2013 is provided between the main absorber 2002 and the elastic member 2012c which lies in the third row as viewed from the outer side. The crotch-side stretched regions 2013 are regions reduced, by stretching, in the basis weight than in other regions. In each crotch-side stretched region 2013, dense portions (reference numeral not given) allowing a small amount of stretching, and coarse portions (reference numeral not given) allowing a larger amount of stretching are provided alternatively in the width direction W of each side-flap portion 2010. Each crotch-side stretched region 2013 is stretched by a factor of stretching ranging from 1.5 to 3.0, as compared with the width before stretching.

Each crotch-side stretched region 2013 has a transparency allowing therethrough visual recognition of internal excretion from the outside. More specifically, the transparency of each crotch-side stretched region 2013 is such as allowing seeing-through of the internal from the outside. The transparency of light elevates by 3% or more by stretching. The internal excretion may visually be recognized from the outside, if the transmissivity of light is 61% or larger (measured conforming to JIS K7105).

Each crotch-side stretched regions 2013 in this embodiment is provided in the width direction W of each side-flap portion 2010, between the main absorber 2002 and the outermost elastic member 2012a. Each crotch-side stretched region 2013 is provided in the longitudinal direction L of the side-flap portion 2010, over the entire range of the crotch region S22. Each crotch-side stretched region 2013 in this embodiment is not extended up to the back-side region S21 nor to the belly-side region S23. A specific method of producing the crotch-side stretched regions 2013 will be detailed later.

The waist flap portion is composed of a pair of back-side waist flap portions 2020 and a pair of belly-side waist flap portions 2021. The pair of back-side waist flap portions 2020 are fixed at one end thereof to the back-side region S21 of the pair of side-flap portions 2010. The back-side waist flap portions 2020 are provided, on the end sides thereof, with bindings 2022.

Each binding 2022 may be a hook-and-loop tightener making use of mechanical force of binding, or an adhesive tape making use of adhesive force. For the case where the bindings 2022 are hook-and-loop tighteners, a material composing the belly-side waist flap portions 2021 needs be a sheet material having a non-woven fabric at least on the surface thereof.

Next, a method of producing the crotch-side stretched regions 2013 will be briefed. The side-flap portions 2010 are obtained by coating a heat-sensitive adhesive (for example, hotmelt adhesive, abbreviated as HMA hereinafter) over a 15-mm-diameter area, so as to adjust the basis weight to 5 g/m² using a spiral spray, and bonding the back sheet 2004 and the exterior sheet 2011. The back sheet 2004 herein is a non-ventilative film having a basis weigh of 23.5 g/m². The exterior sheet 2011 is an SMS having a basis weight of 13 g/m².

A stretching machine 2030 forming the crotch-side stretched regions 2013 has, as illustrated in FIG. 17, a pair of stretching blade array portions 2031 opposed and engageable with each other. Each stretching blade array portion 2031 has a depth of blade of 2.5 mm, a pitch of a pair of blades of 1.25 mm, and the number of blades of 17. The stretching machine 2030 performs stretching while setting the temperature of the stretching blade array portion 2031 to 100°C. By the stretching, the crotch-side stretched regions 2013 may be stretched by a factor of 1.8 as compared with the width before the stretching.

By stretching using the stretching machine 2030, the dense portions allowing a small amount of stretching, and coarse portions allowing a larger amount of stretching are provided to each crotch-side stretched region 2013, alternatively in the width direction W of the side-flap portions 2010.

The amount of stretching is variable by appropriately modifying the above-described conditions. Relative to width W21 of the crotch-side stretched regions 2013 measured before the edge of the center portion of the crotch region S22 of the side-flap portions 2010 is stretched outward (FIG. 18A), width W22 illustrated in FIG. 18B falls in the range from 1.5 to 3.0 times.

Width W22 herein is the width of the crotch-side stretched regions 2013 measured after the edge of the center portion of the crotch region S22 of the side-flap portions 2010 is stretched outward. In short, width W22 is expandable relative to width W21, to as much as a factor of stretching (1.5 to 3.0). Accordingly, the crotch region S22 of the individual side-flap portions 2010 may be stretchable to as largest as a factor of stretching (1.5 to 3.0), as compared with the width dimension of the back-side region S21 and the belly-side region S23.

The transmissivity allowing seeing-through of the internal from the outside may vary depending on types of sheet materials composing the side-flap portions 2010, so that the amount of stretching is adjusted depending on types of the sheet materials, so as to obtain a desired transparency allowing seeing-through of the internal from the outside.

When thus-configured diaper 2001A is worn by the user, as illustrated in FIG. 16, the outer periphery of each side-flap portion 2010 comes into contact with the leg-opening portion of the user as a result of shrinkage of the elastic members 2012a, 2012b. In addition, a space, or so-called pocket P, is formed in the crotch side of the user, because the expandable width dimension of the individual side-flap portions 2010 in the crotch region S22 is larger than the width dimensions of the side-flap portions 2010 in the back-side region S21 and the belly-side region S23, by contribution of the pair of crotch-side stretched regions 2013.

The individual crotch-side stretched regions 2013 may be formed by stretching the individual side-flap portions 2010 in the crotch region S22 in the width direction W. Accordingly, the crotch-side stretched regions 2013 may be formed using a material having only the same width with those in a diaper used without forming so-called pocket P in the crotch region S22.

Additionally, in the diaper 2001A, the pocket P may be formed on the crotch side of the user, without providing the fold-back portions to the individual side-flap portions 2010, and without providing the elastic members 2012 in a curved pattern to the individual side-flap portions 2010, unlike the conventional example. As a consequence, the diaper 2001A may reduce the material consumption, simplify processes in the manufacturing, and reduce the cost of manufacturing.

Because portions of the individual side-flap portions 2010, covering the leg-opening portion close to the crotch of the user, are given as thin and soft crotch-side stretched regions, the diaper 2001A may raise advantages in that it may be less uncomfortable in wearing, it may ensure high compliance with motion of the legs of the user, it may ensure easy rise-up from the main absorber, and it may be brought into close contact with the leg-opening portion without too much tightening the outer peripheries of the individual side-flap portions 2010.

Since each crotch-side stretched region 2013 is provided also in the region more closer to the main absorber 2002 away from the width bisector of the portion between each side edge of the main absorber 2002 and the outermost elastic member 2012a, so that this embodiment raises also advantages described below. The diaper 2001A worn by the user goes to bend at portions where difference in rigidity occurs, that is, at the boundary positions between the main absorber 2002 and the individual side-flap portions 2010. In this embodiment, these portions are configured by the flexible crotch-side stretched regions 2013, so that the diaper 2001A may be excellent in the rise-up performance, and may ensure easy formation of so-called pocket P.

The diaper 2001A of this embodiment has the back-side waist flap portions 2020 and the belly-side waist flap portions 2021, provided so as to swell out from both sides of the back-side region S21 and the belly-side region S23 of the pair of side-flap portions 2010. The individual crotch-side stretched regions 2013 are provided only in the range of the crotch region S22. Accordingly, when the diaper 2001A is worn by the user making use of the back-side waist flap portions 2020 and the belly-side waist flap portions 2021, the force of tightening of the back-side waist flap portions 2020 and the belly-side waist flap portions 2021 is not directly exerted on the crotch-side stretched regions 2013. Therefore, formation of the pocket in the crotch region S22 of the diaper 2001A is not inhibited, and the diaper 2001A is not reduced in the strength.

Because the individual crotch-side stretched regions 2013 in this embodiment have a transparency allowing therethrough visual recognition of internal excretion from the outside, so that excretion in the diaper 2001A may visually be confirmed from the outside through the crotch-side stretched regions 2013. The diaper 2001A thus allows easy judgment of exchange of the diaper.

In this embodiment, each crotch-side stretched region 2013 has dense portions allowing a small amount of stretching and coarse portions allowing a larger amount of stretching, provided alternatively in the width direction W of each side-flap portion 2010. Accordingly, in the process of manufacturing the diaper 2001A, each crotch-side stretched region 2013 is alternately folded by the dense portions and the coarse portions, so that the outer edge of each side-flap portion 2010 may be straight without causing projection. The diaper 2001A may, therefore, be easy to handle typically in the processes of manufacturing and packaging of the products.

In this embodiment, each crotch-side stretched region 2013 is stretched by a factor of 1.5 to 3.0 as compared with the width before the stretching. The factor of stretching of the crotch-side stretched regions 2013 smaller than 1.5 may result in only a small height of rise-up of the side-flap portions 2010, so that the elastic members 2012 may undesirably fail in establishing close contact with the skin. On the other hand, the factor of stretching of the crotch-side stretched regions 2013 exceeding 3.0 may heavily damage the material composing the side-flap portions 2010 due to stretching, and may raise a risk of fractures of the materials, such as breakage and piercing.

Accordingly, by adjusting the factor of stretching of the crotch-side stretched regions 2013 within the range from 1.5 to 3.0, a space, or so-called pocket, may surely be formed in the crotch region S22 of the diaper 2001A, without damaging the materials.

### (Modified Examples of Embodiment 2-1)

Although the side-flap portions 2010 in embodiment 2-1 were composed of two layers, which were the exterior sheet 2011 and the back sheet 2004, the individual side-flap portions 2010 may be configured only by the back sheet 2004. By configuring the side-flap portions 2010 using a single layer of the exterior sheet 2011, consumption of the exterior sheet 2011 may be reduced.

Alternatively, the side-flap portions 2010 may be configured by three layers, which are the exterior sheet 2011, the back sheet 2004 and the top sheet 2003. By configuring the side-flap portions 2010 using three layers of the exterior sheet 2011, the back sheet 2004 and the top sheet 2003, the strength of the crotch-side stretched regions 2013 in the individual side-flap portions 2010 may be increased.

The back sheet 2004 may be also such as being composed of different sheet materials for the crotch-side stretched regions 2013 and for the residual regions. For example, by using a sheet material suitable for stretching for the crotch-side stretched region 2013, and by using a sheet material suitable as the back sheet of the main absorber 2002, the back sheet 2004 may be made excellent both in the stretchability and liquid impermeability.

### (Embodiment 2-2)

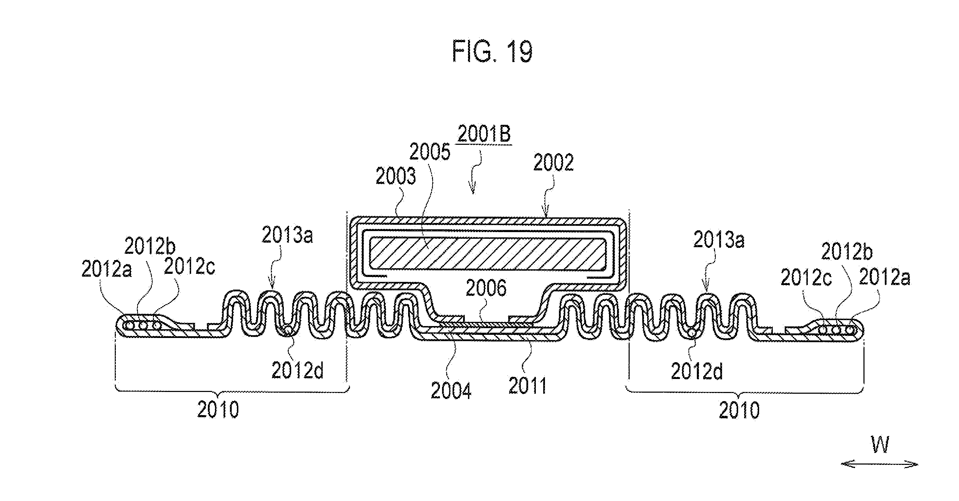

FIG. 19 is a sectional view of a diaper 2001B according to embodiment 2-2 of the present invention.

As illustrated in FIG. 19, the diaper 2001B according to embodiment 2-2 differs from the above-described embodiment 2-1, in the configurations of the main absorber 2002 and the crotch-side stretched regions 2013a in the individual side-flap portions 2010. More specifically, the top sheet 2003 and the back sheet 2004 of the main absorber 2002 are bonded by the bonding portion 2006, only at the center portion in the width direction W, while leaving both end portions unbonded. Each crotch-side stretched region 2013a is provided so as to extend up to a region where it overlaps the main absorber 2002 (unbonded portion).

Other aspects of the configuration are same with those in the above-described embodiment 2-1, so that the explanation therefor will not be repeated, while giving instead the same reference numerals to the same components in the drawings aiming at clarity.

Since each crotch-side stretched region 2013a in this embodiment is provided so as to extend up to a region where it overlaps the main absorber 2002, the crotch-side stretched region 2013a may be made larger in the width dimension, and thereby the diaper 2001B may form a space, or so-called pocket, having a large depth in the crotch side thereof.

### (Embodiment 2-3)

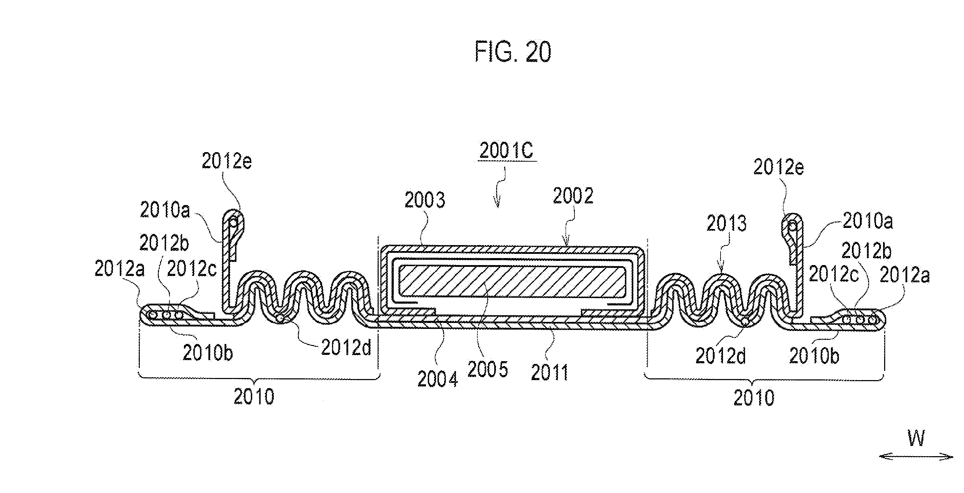

FIG. 20 is a sectional view of a diaper 2001C according to embodiment 2-3 of the present invention.

As illustrated in FIG. 20, the diaper 2001C according to embodiment 2-3 differs from the above-described embodiment 2-1, only in the configuration of the individual side-flap portions 2010. Each side-flap portion 2010 branches, at a portion outside of each side-flap portion 2010 in the width direction W, into two ways, which are an inner flap portion 2010a and an outer flap portion 2010b. A soft-touch sheet material is used for the inner flap portions 2010a. In the end portions of both of the inner flap portions 2010a and the outer flap portions 2010b, there are provided the elastic members 2012a, 2012b, 2012c and 2012e, under a stretched state. As a consequence, when the diaper 2001C is worn by the user, the side-flap portions 2010 doubly come into contact with the leg-opening portion, so that the diaper 2001C may more surely prevent the side leakage. The diaper 2001C is also excellent in the touch, because the inner flap portions 2010a are composed of a soft-touch sheet material.

Other aspects of the configuration are same with those in the above-described embodiment 2-1, so that the explanation therefor will not be repeated, while giving instead the same reference numerals to the same components in the drawings aiming at clarity.

### (Modified Example of Embodiment 2-3)

Although, in embodiment 2-3, the elastic members 2012a, 2012b, 2012c and 2012e were provided to the end portions of both of the inner flap portion 2010a and the outer flap portion 2010b, the diaper 2001C may be provided with the elastic members stretched, only to the end portion of the inner flap portion 2010a. The diaper 2001C in this case is given as so-called, inwardly-inclined, three-dimensionally gathered configuration.

### (Embodiment 2-4)

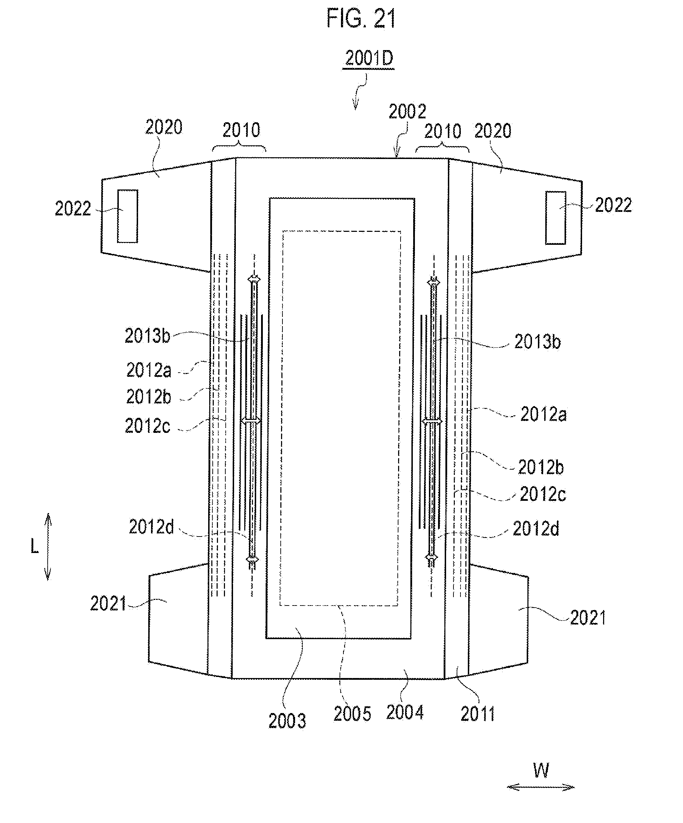

FIG. 21 is an expanded view illustrating a diaper 2001D according to embodiment 2-4 of the present invention.

As illustrated in FIG. 21, the diaper 2001D according to embodiment 2-4 differs from the above-described embodiment 2-1, only in the configuration of the individual crotch-side stretched regions 2013b. A width dimension, under a stretched state, of the stretchable portion thereof at the center position in the longitudinal direction L in the crotch region is larger than a width dimension of the stretchable portions on both ends in the longitudinal direction L of each side-flap portion 2010. This is because the crotch-side stretched regions 2013b have different width dimensions over which the stretching is applied, when viewed along the longitudinal direction L. More specifically, the width dimension of the stretchable portion at the center position in the longitudinal direction L is set longer than the width dimension of the stretchable portions at both ends in the longitudinal direction L.

The diaper 2001D can form a pocket having a small rise-up dimension in the edge portions, ascribable to restriction of the edge portions of the crotch-side stetched regions 2013b by the portions other than the crotch-side stretched regions 2013b, and having a largest rise-up dimension at the center portion. The crotch-side stretched regions 2013b therefore be loss-less in the pocket formation.

Alternatively, the crotch-side stretched regions 2013b may be provided so as to have the same width dimension over which the stretching is applied as viewed along the longitudinal direction L, but have different amount of stretching per unit length, to thereby allow the stretchable portions at the center position in the longitudinal direction L to have larger width dimension when stretched (in a state of wearing), as compared with the stretchable portion at both ends in the longitudinal direction L.

### (Embodiment 2-5)

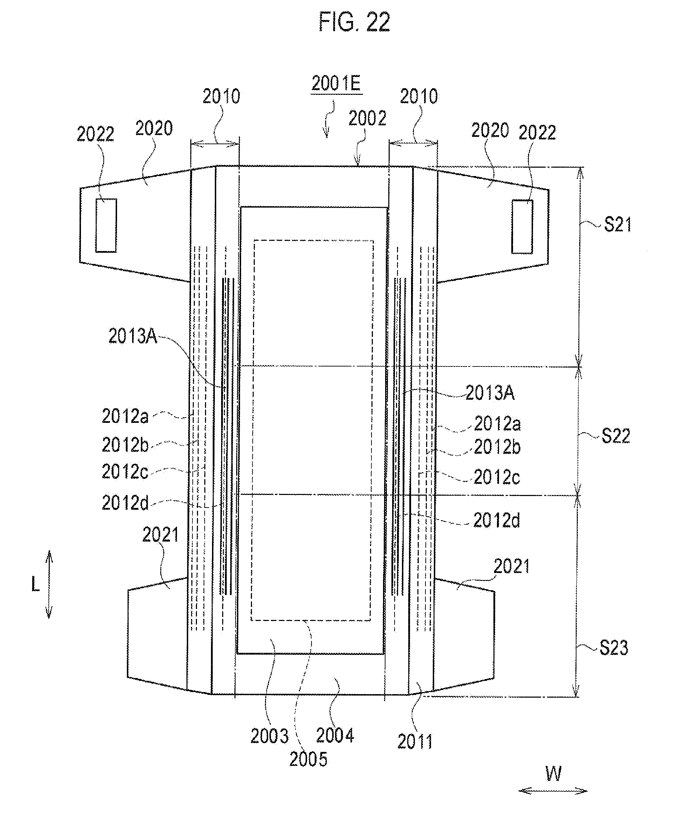

FIG. 22 is a sectional view of a diaper 2001E according to embodiment 2-5 of the present invention.

As illustrated in FIG. 22, the diaper 2001E according to embodiment 2-5 is different from the above-described embodiment 2-1, only in the configuration of a pair of crotch-side stretched regions 2013A. Each crotch-side stretched region 2013A is not only provided in the crotch region S22 of each side-flap portion 2010, but also extended up to both of the back-side region S21 and the belly-side region S23. The end portions of the crotch-side stretched regions 2013A on the back side and the end portions thereof on the belly side fall short of the edge of the side-flap portions 2010 on the back side and the edge of thereof on the belly side.

Since, in this embodiment, a space, or so-called pocket, formed in the diaper 2001E may be formed not only in the crotch region S22, but also in the back-side region S21 and the belly-side region S23, so that the diaper 2001E can temporarily retain a large amount of excretion, and can more surely prevent side leakage of excretion. The diaper 2001E is particularly effective in prevention of side leakage of both of feces and urine.

### (Individual Modified Examples of Embodiment 2-5)

One possible modified example of embodiment 2-5 may be such as providing each crotch-side stretched region 2013A extended from the crotch region S22 of each side-flap portion 2010 only towards the back-side region S21. According to this modified example, a space, or so-called pocket, is formed in the diaper not only in the crotch region S22, but also in the back-side region S21, so that the diaper is particularly effective in prevention of side leakage of feces.

Another possible modified example of embodiment 2-5 may be such as providing each crotch-side stretched region 2013A extended from the crotch region S22 of each side-flap portion 2010 only towards the belly-side region S23. According to this modified example, a space, or so-called pocket, is formed in the diaper not only in the crotch region S22, but also in the belly-side region S23, so that the diaper is particularly effective in prevention of side leakage of urine.

### (Others)

Although in the above-described embodiments, each side-flap portion 2010 was provided with four rows of elastic members 2012a to 2012d, the elastic members may also be provided to each side edge of the side-flap portion 2010 only in a single row or two rows. The number of elastic members and position of arrangement may appropriately be modified.

Although the above embodiments explained the case where the present invention was applied to the open-type diapers, the present invention is similarly applicable also to the pants-type diapers. The present invention is applicable still also to any absorptive articles other than the diapers.

This application is based upon and claims the benefit of priority from the prior Japanese Patent Application No. JP 2008-037916, filed on February 19, 2008; the entire contents of which are incorporated herein by reference.

### INDUSTRIAL APPLICABILITY

As has been described in the above, the absorptive article of the present invention can prevent permeation and leakage, and is excellent in ventilation. The absorptive article of the present invention is also such as forming so-called pocket on the crotch side of the user, and can reduce material consumption, simplify processes in the manufacturing, and reduce the cost of manufacturing. The present invention is effectively applicable to absorptive article such as diaper.

## Claims

1. An absorptive article comprising:
a main absorber composed of a liquid-permeable top sheet, liquid-impermeable back sheet, and an absorber disposed between the top sheet and the back sheet, absorbing and retaining fluid, and including a back-side region, a crotch region, and a belly-side region arranged in the longitudinal direction from the back side to the belly side in a state of wearing; and
a left-and-right pair of side-flap portions provided on both sides in the width direction of the main absorber, wherein
the lower surface of the main absorber and the back sheet located at the side-flap portions are joined, and
each side-flap portion has a highly-moisture-permeable region having a larger moisture permeability as compared with the lower surface of the main absorber, between the width edge of each side-flap portion and each side edge of the absorber.

2. The absorptive article according to claim 1, wherein
the back sheet is formed by a micro-porous, liquid-impermeable film composed of a resin containing an inorganic material as a filler, and
the highly-moisture-permeable region is formed in the liquid-impermeable film.

3. The absorptive article according to claim 1, wherein
the back sheet is formed by adhering the liquid-impermeable sheet and an exterior sheet,
the liquid-impermeable sheet is formed by a micro-porous, liquid-impermeable film composed of a resin containing an inorganic material as a filler, and
the highly-moisture-permeable region is formed in the liquid-impermeable sheet and the exterior sheet.

4. The absorptive article according to claim 1, wherein
the back sheet is formed by adhering the liquid-impermeable sheet and an exterior sheet,
the liquid-impermeable sheet is formed by a micro-porous, liquid-impermeable film composed of a resin containing an inorganic material as a filler, and
the highly-moisture-permeable region is formed in the liquid-impermeable sheet, the exterior sheet, and the top sheet.

5. The absorptive article according to claim 1, wherein
the side-flap portions in the back-side region are provided on both sides thereof in the width direction with a left-and-right pair of waist flap portions extended from both side edges of the side-flap portions, and
the waist flap portions are provided on both sides in the width direction , with a pair of bindings bindable with the liquid-impermeable back sheet in the belly-side region in a state of wearing.

6. The absorptive article according to claim 1, wherein
each side-flap portion has an elastic member disposed stretched, in the longitudinal direction of the main absorber.

7. The absorptive article according to claim 6, wherein
the elastic member is disposed, under a stretched state, to form a gathered portion having a larger moisture permeability as compared with the highly-moisture-permeable region of the side-flap portion.

8. The absorptive article according to claim 1, wherein
the highly-moisture-permeable region is formed by stretching outwardly in the width direction each side-flap portion that located between the edge in the width direction and each side edge of the absorber.

9. The absorptive article according to claim 1, wherein
the highly-moisture-permeable region is set to have a moisture permeability of 1.3 to 2.5 times or more as large as a moisture permeability of the lower surface side of the main absorber, to avoid leakage of fluid.

10. An absorptive article comprising:
a main absorber composed of at least a liquid-permeable top sheet, a liquid-impermeable back sheet, and an absorber disposed between the top sheet and the back sheet, absorbing and retaining fluid, and including a back-side region, a crotch region, and a belly-side region arranged in the longitudinal direction from the back side to the belly side in a state of wearing;
a left-and-right pair of side-flap portions provided on both sides in the width direction of the main absorber; and
a pair of elastic members provided to at least the crotch region of the individual side-flap portions, stretched in the longitudinal direction, further comprising
a crotch-side stretched region stretched in the width direction, provided to the side-flap portion in the crotch region, between the main absorber and the outermost elastic member.

11. The absorptive article according to claim 10, wherein
each crotch-side stretched region is provided, more closer to the main absorber, away from the width bisector between each side edge of the main absorber and the outermost elastic member.

12. The absorptive article according to claim 10, wherein
each crotch-side stretched region is extend from the crotch portion, to at least one of the back-side region and the belly-side region of each side-flap portion.

13. The absorptive article according to claim 10, further comprising
a pair of waist flap portions is swelled outward from at least one of the back-side region and the belly-side region of the pair of side-flap portions.

14. The absorptive article according to claim 10, wherein
each crotch-side stretched region is extended up to a region which overlaps the main absorber.

15. The absorptive article according to claim 10, wherein
each crotch-side stretched region has a transparency allowing therethrough visual recognition of internal excretion from the outside.

16. The absorptive article according to claim 10, wherein
a width dimension ,under a stretched state, of the stretchable portion at the center position in the longitudinal direction in the crotch region is larger than a width dimension of the stretchable portions on both ends in the longitudinal direction of each side-flap portion.

17. The absorptive article according to claim 10, wherein
each crotch-side stretched region has dense portions allowing a small amount of stretching and coarse portions allowing a larger amount of stretching, provided alternatively in the width direction of the side-flap portion.

18. The absorptive article according to claim 10, wherein
each crotch-side stretched region is stretched by a factor of stretching ranging from 1.5 to 3.0.
